(19) Europäisches Patentamt
European Patent Office
Office européen des brevets

(11) Publication number: **0 337 572 B1**

(12) # EUROPEAN PATENT SPECIFICATION

(45) Date of publication of patent specification: **20.01.93 Bulletin 93/03**

(51) Int. Cl.$^5$: **C07C 29/17,** C07C 31/12

(21) Application number: **89200901.0**

(22) Date of filing: **10.04.89**

(54) **Process for the preparation of alkanediols.**

(30) Priority: **14.04.88 GB 8808831**

(43) Date of publication of application: **18.10.89 Bulletin 89/42**

(45) Publication of the grant of the patent: **20.01.93 Bulletin 93/03**

(84) Designated Contracting States: **BE CH DE ES FR GB IT LI NL SE**

(56) References cited:
**JP-A- 5 978 126**
**US-A- 2 992 278**
**US-A- 3 933 919**
**US-A- 4 438 285**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**
Inventor: **Niele, Franciscus Gerardus Maria**
**Badhuisweg 3**
**NL-1031 CM Amsterdam (NL)**

**EP 0 337 572 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for the preparation of alkanediols, having at least two carbon atoms separating the alcohol groups, that means, at least four carbon atoms separating the hydroxy groups. More in particular the process relates to the preparation of said alkanediols via a catalytic hydrogenation of the corresponding alkyne- or alkenediols.

Alkanediols such as butane-1,4-diol, are focussing a lot of attention in view of their high potential for the chemical industry, especially in the preparation of polyurethane foams and other polymers such as polyesters, as well as in the preparation of tetrahydrofuran. In spite of a still growing demand for these alkanediols the use of these alkanediols is still rather restricted due to the problems encountered in their preparation, which generally proceeds via a heterogeneously catalyzed hydrogenation of the corresponding alkynediol. With respect to the preparation of e.g. butane-1,4-diol, it is known that the catalytic hydrogenation of but-2-yne-1,4-diol to butane-1,4-diol proceeds via the formation of the intermediate but-2-ene-1,4-diol, which compound is however known to isomerize to $\gamma$-hydroxy-butyraldehyde thereby decreasing the yield of the desired alkanediols. Although it is possible in principle to hydrogenate $\gamma$-hydroxy-butyraldehyde to butane-1,4-diol, said hydrogenation is considerably more difficult than the hydrogenation of the olefinically unsaturated double bonds. A further problem related to the preparation of the alkanediol, as described hereinbefore is, that the catalysts which are effective in the hydrogenation of the unsaturated groups, e.g. noble metals and especially palladium (Pd), are frequently also effective isomerization catalysts, i.e. they promote the formation of compounds such as $\gamma$-hydroxy-butyraldehyde.

In US-A-4438285 a process is described for the catalytic hydrogenation of but-2-yne-1,4-diol to butane-1,4-diol, which process is conducted at a temperature of 60-180 °C, preferably at a temperature of 110 °C, wherein the catalyst comprises metallic ruthenium and palladium in a 4:1 m ratio supported on a carrier, the ruthenium being present in order to catalyze the hydrogenation of any gamma-hydroxy-butyraldehyde formed. This process has a disavantage in that it employs expensive ruthenium in large amounts.

US-A-2992278 discloses a process for the hydrogenation of tertiary acetylenic 1,4-diols having tertiary alcohol groups, wherein a metallic palladium catalyst is used. An alkaline material is added for suppressing any undesired hydrogenolysis side reaction typically occuring at tertiary hydroxy groups.

In British Patent GB-2 058 074-B a process is described for a homogeneously catalyzed hydrogenation of olefinically unsaturated compounds, such as alkenes and styrene, in the presence of a palladium complex of general formula $[Pd(L_3YL_4)(\text{donor solvent})_2][X]_2$, wherein the donor solvents are weakly co-ordinated solvento-ligands and $L_3$-Y-$L_4$ is a bidentate ligand containing groups $L_3$ and $L_4$, each of which has a Group V or Group VI donor atom attached to the palladium atom, $L_3$-Y-$L_4$ being of the form either $R_1R_2E_1$-Y-$E_2R_3R_4$ or $R_1E_3$-Y-$E_4R_2$ wherein each of $R_1$, $R_2$, $R_3$ and $R_4$ is an optionally substituted alkyl or aryl group; wherein Y is either $(CH_2)_n$ in which n is 1, 2 or 3, or o-phenylene, or cis-vinylene, and wherein each of $E_1$ and $E_2$ is selected from nitrogen, phosphorus or arsenic and each of $E_3$ and $E_4$ is selected from oxygen, sulphur or selenium, and X is a non-coordinating anion. It is not suggested that these catalysts would be active for the hydrogenation of alkyne- or alkenediols.

From the experimental data provided it can be concluded that said catalyst is selective, but not very active for hydrogenation reactions. Not only is the catalyst employed in a relatively high concentration, but moreover the reaction time is relatively long. As these rather mediocre results were obtained with relatively simple, unsaturated compounds i.e. oct-1-ene and styrene, it can hardly be expected that their activity for the hydrogenation of considerably more complex, unsaturated compounds such as but-2-yne-1,4-diol, would be better.

Hence it can be concluded that there is room for further improvement in the preparation of alkanediols via a catalytic hydrogenation of the corresponding alkyne- and/or alkenediols.

The object of the present invention is to provide a process for the preparation of alkanediols, which does not present the problems hereinbefore described.

As a result of continuing and extensive research and experimentation, it has now surprisingly been found that it is possible to prepare alkanediols of the before mentioned type in a high yield and with high selectivity, by a catalytic hydrogenation process which is carried out under mild reaction conditions and in the presence of specific cationic palladium compounds and a bidentate ligand.

The invention provides therefore a process for the preparation of alkanediols having at least two carbon atoms separating the alcohol groups, which comprises the reaction of

a) an alkynediol and/or an alkenediol of which the alcohol groups are separated by at least two carbon atoms linked by the alkynic or alkenic bond respectively, and

b) hydrogen

in the presence of a catalyst composition based upon a compound of cationic palladium, a co-ordinating anion and a bidentate ligand of the general formula $R^1R^2$-M-R-M-$R^3R^4$, wherein M represents phosphorus, arsenic

or antimony, R is a bivalent organic bridging group containing from two to four carbon atoms in the bridge and $R^1$, $R^2$, $R^3$ and $R^4$ represent hydrocarbyl groups which may or may not be substituted with polar groups.

The preparation of alkanediols via the process of the present invention may conveniently be carried out in the presence of a solvent or diluent; preferably the solvent or diluent is a polar organic compound, thereby providing a polar reaction medium.

The nature of the polar reaction medium is not critical and may comprise one or more liquid polar organic compounds, as well as mixtures thereof with water. It will be appreciated by persons skilled in the art that it would be advantageous to use a polar reaction medium which is inert with respect to the hydrogenation reaction, i.e. which does not interfere with the desired reaction. Suitable polar organic compounds include linear and cyclic mono- and polyethers such as ethylene glycol dimethyl ether, ethylene glycol diethyl ether, diethylene glycol dimethyl ether (diglyme), diethylene glycol diethyl ether, triethylene glycol dimethyl ether, tetrahydrofuran, dioxan, diphenylether, ethylene glycol monomethyl ether, ethylene glycol monoethyl ether, ethylene glycol monobutyl ether, diethylene glycol monomethyl ether, diethylene glycol monoethyl ether, diethylene glycol monopropyl ether, propylene glycol monomethyl ether, 1,3-propanediol monomethyl ether and the like. Alcohols, such as those whereon the alkali or alkaline-earth metal alcoholates as described hereinafter are based, as well as the starting diols and the alkanediols prepared by the process of the present invention may also serve as a polar solvent. Diglyme is a preferred polar organic compound for use as polar reaction medium.

Compounds of cationic palladium which may conveniently be employed in the process of the present invention are preferably based on a co-ordinating anion and include palladium salts based on acids such as halohydrogen acids, e.g. hydrochloric acid, carboxylic acids having up to 12 carbon atoms and phosphorus based acids. Palladium carboxylates are preferred palladium compounds, palladium acetate being a preferred palladium carboxylate.

The bidentate ligands which are preferred in the catalyst composition used in the process of the present invention are those wherein the groups $R^1$, $R^2$, $R^3$ and $R^4$ represent aryl groups having from 6-14 carbon atoms. Phenyl groups are preferred such aryl groups. The bridging group R which is present in the bidentate ligands, as described hereinbefore, may be selected from the groups comprising: $-(CH_2)_n-$ wherein n is 2, 3 or 4; $-CH_2-C(CH_3)(CH_2MR^5R^6)-CH_2-$ wherein $R^5$ and $R^6$ have the same meanings as $R^1$, $R^2$, $R^3$ and $R^4$ and M has the same meaning as hereinbefore; o-phenylene and cis-vinylene. A preferred such bridging group is $-(CH_2)_3-$. In the ligands as described hereinbefore it is preferred that in the general formula M represents phosphorus. 1,3-bis(diphenylphosphino)propane is a preferred phosphorus containing bidentate ligand.

In the catalyst composition employed in the process of the present invention the cationic palladium compounds will generally be employed in a ratio of from 0.1-10 moles per mol of ligand, and preferably in the range of from 0.5-5 moles per mol of ligand.

One of the advantages of the process of the present invention over that of the prior art is that it may use palladium concentrations which are very low relative to those of the compounds to be hydrogenated, i.e. the unsaturated diol.

The process of the present invention may conveniently be conducted at a temperature in the range of from 15-125 °C and a hydrogen pressure in the range of from atmospheric pressure to 100 bar.

In a preferred embodiment of the process of the present invention the catalyst composition comprises, in addition to the palladium compound and the ligand, also a base. The presence of a base in the catalyst composition has proven to be advantageous in that it can reduce the reaction time and/or increase the selectivity towards the desired alkanediol even further.

The base which is employed in the preferred embodiment of the process of the present invention may be an organic or an inorganic base.

Organic bases which may suitably be employed in the process of the present invention include alkali metal, alkaline-earth metal or ammonium alcoholates based on linear or branched aliphatic alcohols, as opposed to primary, secondary and tertiary amines, which were surprisingly found to be ineffective. Preferably such alcohols have 1-8 carbon atoms per molecule. Especially preferred are branched aliphatic alcohols such as tertiary alcohols; tert-butyl alcohol and tert-amyl alcohol being preferred tertiary alcohols. Alkali metal alcoholates based on the tertiary alcohols as described hereinbefore, are preferred alcoholates; potassium tert-butylate and sodium tert-amylate being especially preferred.

It will be appreciated by the skilled reader that when the process of the present invention is conducted in the presence of water and an alcoholate, as hereinbefore described, that as a result of the interaction of water and alcoholate, the corresponding hydroxide will be formed. Hence, when the preparation of the alkanediols is conducted in a water-containing reaction medium, the alcoholate may advantageously be replaced with an inorganic base. Inorganic bases which may be used in a water-containing reaction medium include alkali metal, alkaline-earth metal and ammonium hydroxides. Potassium hydroxide is a preferred alkali metal hydroxide.

In the process of the present invention it is preferred that the base is employed in a molar excess over the

palladium compound. Especially preferred is a process wherein the molar ratio of base to palladium compound is in the range of from 2:1 to 1000:1.

When an inorganic base, as described hereinbefore, is employed in the process of the present invention, said base may conveniently be added to the reaction medium as an aqueous solution.

In the process of the present invention it has been found possible to employ a precursor of the palladium compound based on a co-ordinating anion, i.e. a palladium compound based on a non-coordinating anion, when a base as described hereinbefore is also present. Without wishing to be bound by any theory it is believed that as a result of an interaction between said base and the precursor, the corresponding palladium compound based on a co-ordinating anion is formed, i.e. a palladium alcoholate or palladium hydroxide. In this embodiment of the present invention the coformulation of a base may afford the in situ preparation of the palladium compound based on a co-ordinating anion.

As mentioned hereinbefore, the starting compounds for the preparation of the alkanediols, via the process of the present invention, are monoalkynically or monoalkenically unsaturated diols, wherein the alcohol groups are separated by at least two carbon atoms. The present process is especially suited for the preparation of alkanediols wherein the alcohol groups are separated by only two carbon atoms. Preferred alkynediols and alkenediols to be used for the preparation of alkanediols wherein the alcohol groups are separated by only two carbon atoms, are those of general formulae (1) and (2)

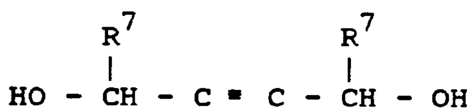
(1)

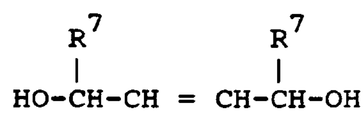
(2)

respectively, wherein each pair of $R^7$ is H or $CH_3$.

The unsaturated diols to be used in the present process will generally be undiluted. However, it may sometimes be possible or even advantageous to employ such an unsaturated diol in diluted form, provided the diluent does not interfere with the process, i.e. the diluent is inert under the reaction conditions of the present process. An example of the use of a diluted unsaturated diol is the use of but-2-yne-1,4-diol as a starting material, which diol is available as an aqueous effluent from the reaction between aqueous formaldehyde and acetylene.

But-2-yne-1,4-diol is a preferred alkynediol for use as a starting material in the process of the present invention.

The unsaturated diol will generally be employed in such an amount that it comprises 5 to 75% m of the reaction medium, although higher and lower concentrations are not excluded.

Preferably the process is conducted at a temperature in the range of from 20-70° C and at a $H_2$ pressure in the range of from 5-70 bar.

The invention will be further illustrated by the following examples for which the following information is provided:

In all examples 1,3-bis(diphenylphosphino)propane was employed as the bidentate ligand which compound will be referred to as BDL.

All reactions were stopped, for the sake of convenience, after the times as indicated in the examples hereinafter. The quoted reaction times should thus not be construed to be the optimum reaction times. Extension of the reaction time, when appropriate, will further increase the conversion and/or selectivity.

<u>Example I</u>

0.1 mmol palladium acetate, 0.15 mmol BDL and 50 ml diglyme were introduced into a 250 ml stainless steel autoclave, equipped with a magnetic stirrer and stirred for a few minutes at 45 °C. This was followed by the addition of 10 g but-2-yne-1,4-diol and pressurizing the autoclave with $H_2$ until a $H_2$ pressure of 60 bar had been obtained. Subsequently the reactor contents were stirred at 45 °C for 5 hours. After cooling the reactor contents were left overnight under $H_2$ pressure, which was followed by depressurizing and analyzing the reactor contents by gas liquid chromatography.

Process details and product analysis data are given in Table 1.

Example II

The procedure as described in Example I was repeated employing the same compounds but in different amounts, while furthermore the reaction time was reduced to 0.5 hours.

Example III

The procedure as described in Example I was repeated, but with twice the amount of diglyme and 1 mmol of potassium tert-butylate, which compound was introduced together with the diglyme. Furthermore the reaction time was reduced to 0.5 hours.

Example IV

The procedure as described in Example III was repeated with twice the amount of BDL and 5 times the amount of potassium tert-butylate, and a doubled reaction time.

Example V

This example was carried out to demonstrate the beneficial effect of potassium tert-butylate when a non-coordinating acid or the anion thereof is present in the reaction medium.

0.1 mmol palladium acetate, 2 mmol of p-toluene sulfonic acid, 0.15 mmol BDL and 100 ml diglyme were introduced into the autoclave and stirred for a few minutes. This was followed by the addition of 5 mmol potassium tert-butylate and 10 g but-2-yne-1,4-diol. From here on the procedure was similar to that of the examples hereinbefore.

Example VI

This example was carried out to demonstrate the possibility of preparing butane-1,4-diol in a water-containing reaction medium and employing KOH as base.

Diglyme was heated in the autoclave in a nitrogen atmosphere to 60 °C. Subsequently the heating was switched off and palladium acetate and BDL were added to the reactor. After mixing the reactor contents for about 15 minutes the diol was added, which was followed after another 20 minutes by the addition of KOH and water and heating the reactor contents to 50 °C. Next the reactor was pressurized with $H_2$ to obtain a pressure of 50 bar and heating continued for the time as indicated hereinafter. Process details and product analysis data are given in Table 1.

Example VII

The procedure as described for Example V was repeated with the difference that the addition of the potassium tert-butylate was omitted.

The process details and product analysis data have been included in Table 1.

From the results of Examples I-VII it can be seen that by the process of the present invention butane-1,4-diol can be formed under mild conditions in high yield and with a high selectivity, while using only very low amounts of catalyst with respect to the diol. The beneficial influence of the presence of a base is also clearly demonstrated, by comparing the results of Examples V and VII, or those of Examples III/IV and II.

Table 1

| Example No. | | I | II | III | IV | V | VI | VII |
|---|---|---|---|---|---|---|---|---|
| Pd. acetate | mmol | 0.1 | 0.1 | 0.1 | 0.1 | 0.1 | 0.1 | 0.1 |
| BDL | mmol | 0.15 | 0.3 | 0.15 | 0.3 | 0.15 | 0.11 | 0.15 |
| Potassium tert-butylate | mmol | - | - | 1 | 5 | 5 | - | - |
| KOH | mmol | - | - | - | - | - | 5 | - |
| p-toluenesulfonic acid | mmol | - | - | - | - | 2 | - | 2 |
| diglyme | ml | 50 | 100 | 100 | 100 | 100 | 50 | 100 |
| but-2-yne-1,4-diol | g | 10 | 10 | 10 | 10 | 10 | 10 | 10 |
| $H_2O$ | ml | - | - | - | - | - | 10 | - |
| $P_{H2}$ | bar | 60 | 60 | 60 | 60 | 60 | 50 | 60 |
| heating | h at °C | 5-45 | 0.5-45 | 0.5-45 | 1-45 | 1-45 | 12-52 | 1-45 |
| but-2-yne-1,4-diol | %m | 100 | 100 | 100 | 100 | 100 | 100 | 95 |
| conversion | | | | | | | | |
| butane-1,4-diol | %m | 92 | 87 | 94 | 99 | 99 | 89 | 69 |
| content of end product | | | | | | | | |
| γ-hydroxy-butyraldehyde | %m | 8 | 13 | 6 | 1 | 1 | 11 | - |
| but-2-ene-1,4-diol | %m | - | - | - | - | - | - | 26 |
| content of end product | | | | | | | | |

## Claims

1. Process for the preparation of alkanediols having at least two carbon atoms separating the alcohol groups, which comprises the reaction of
   a) an alkynediol and/or an alkenediol, of which the alcohol groups are separated by at least two carbon atoms linked by the alkynic or alkenic bond respectively, and
   b) hydrogen,
   in the presence of a catalyst composition based upon a compound of cationic palladium, a co-ordinating anion and a bidentate ligand of the general formula $R^1R^2$-M-R-M-$R^3R^4$, wherein M represents phosphorus, arsenic or antimony, R is a bivalent organic bridging group containing from two to four carbon atoms in the bridge and $R^1$, $R^2$, $R^3$ and $R^4$ represent hydrocarbyl groups which may or may not be substituted with polar groups.

2. A process as claimed in claim 1, wherein the groups $R^1$, $R^2$, $R^3$ and $R^4$ represent aryl groups having from 6-14 carbon atoms.

3. A process as claimed in claim 2, wherein the aryl groups are phenyl groups.

4. A process as claimed in any of claims 1-3, wherein the bridging group R is selected from the group comprising: $-(CH_2)_n-$ wherein n is 2, 3 or 4: $-CH_2-C(CH_3)(CH_2MR^5R^6)-CH_2-$ wherein $R^5$ and $R^6$ have the same meanings as $R^1$, $R^2$, $R^3$ and $R^4$ and M has the meaning defined in claim 1; o-phenylene and cis-vinylene.

5. A process as claimed in any of claims 1-4, wherein M represents phosphorus.

6. A process as claimed in claims 3, 4 and 5, wherein the ligand is 1,3-bis(diphenylphosphino)propane.

7. A process as claimed in any of claims 1-6, wherein the palladium compound is based on a coordinating anion, such as an acetate.

8. A process as claimed in any of claims 1-7, wherein the palladium compound is employed in a ratio in the range of 0.1-10 moles per mol of ligand.

9. A process as claimed in any of claims 1-8, wherein the palladium compound is present in a ratio in the range of $10^{-7}$-$10^{-2}$ moles per mol of unsaturated diol.

10. A process as claimed in any of claims 1-9, wherein in addition to the palladium compound and the ligand the catalyst composition comprises a base.

11. A process as claimed in any of claims 1-10, wherein the process is conducted at a temperature in the range of from 15-125 °C and a hydrogen pressure in the range of from atmospheric pressure to 100 bar.

12. A process as claimed in any of claims 1-11, wherein the process is conducted in a polar medium, such as diethylene glycol dimethyl ether.

## Patentansprüche

1. Verfahren zur Herstellung von Alkandiolen, bei denen die Alkoholgruppen durch mindestens zwei Kohlenstoffatome voneinander getrennt sind, umfassend die Reaktion von
   a) einem Alkyndiol und/oder Alkendiol, bei dem die Alkoholgruppen durch mindestens zwei Kohlenstoffatome voneinander getrennt sind, die ihrerseits mittels einer Alkynyl- oder Alkenylbindung miteinander verbunden sind, und
   b) Wasserstoff
   in Anwesenheit einer katalytischen Zusammensetzung auf der Basis einer Verbindung von kationischem Palladium, eines koordinierenden ànions und eines zweizähnigen Liganden der allgemeinen Formel $R^1R^2$-M-R-M-$R^3R^4$, in der M Phosphor, Arsen oder Antimon bedeutet, R eine zweiwertige organische Brückengruppe darstellt, die zwei bis vier Kohlenstoffatome in der Brücke enthält, und $R^1$, $R^2$, $R^3$ und $R^4$ jeweils Kohlenwasserstoffgruppen sind, die gegebenenfalls mit polaren Gruppen substituiert sind.

2. Verfahren, wie in Anspruch 1 beansprucht, in dem die Gruppen $R^1$, $R^2$, $R^3$ und $R^4$ Arylgruppen mit 6 bis

14 Kohlenstoffatomen darstellen.

3. Verfahren, wie in Anspruch 2 beansprucht, in dem die Arylgruppen Phenylgruppen sind.

4. Verfahren, wie in irgendeinem der Ansprüche 1 bis 3 beansprucht, in dem die Brückengruppe R ausgewählt ist aus der Gruppe umfassend: -(CH2)n-, wobei n den Wert 2, 3 oder 4 hat; $-CH_2-C(CH_3)(CH_2MR^5R^6)-CH_2-$, wobei $R^5$ und $R^6$ die gleiche Bedeutung haben, wie für $R^1$, $R^2$, $R^3$ und $R^4$ angegeben, und M die Bedeutung hat, wie in Anspruch 1 definiert ist; o-Phenylen und cis-Vinylen.

5. Verfahren, wie in irgendeinem der Ansprüche 1 bis 4 beansprucht, in dem M Phosphor bedeutet.

6. Verfahren, wie in Anspruch 3, 4 und 5 beansprucht, in dem der Ligand die Verbindung 1,3-Bis(diphenylphosphino)propan ist.

7. Verfahren, wie in irgendeinem der Ansprüche 1 bis 6 beansprucht, in dem die Palladiumverbindung auf einem koordinierenden Anion, wie einem Azetat, basiert.

8. Verfahren, wie in irgendeinem der Ansprüche 1 bis 7 beansprucht, in dem die Palladiumverbindung in einem Bereich von 0,1 bis 10 Mol je Mol Ligand angewendet wird.

9. Verfahren, wie in irgendeinem der Ansprüche 1 bis 8 beansprucht, in dem die Palladiumverbindung in einem Bereich von 10-7 bis $10^{-2}$ Mol je Mol ungesättigtes Diol vorliegt.

10. Verfahren, wie in irgendeinem der Ansprüche 1 bis 9 beansprucht, in dem die katalytische Zusammensetzung zusätzlich zur Palladiumverbindung und dem Liganden auch noch eine Base umfaßt.

11. Verfahren, wie in irgendeinem der Ansprüche 1 bis 10 beansprucht, wobei das Verfahren bei einer Temperatur im Bereich von 15 bis 125 °C und bei einem Wasserstoffdruck im Bereich von Atmosphärendruck bis 100 Bar durchgeführt wird.

12. Verfahren, wie in irgendeinem der Ansprüche 1 bis 11 beansprucht, wobei das Verfahren in einem polaren Medium, wie Diethylenglycoldimethylether, durchgeführt wird.

## Revendications

1. Procédé de préparation d'alcanediols comportant au moins deux atomes de carbone qui séparent les groupes alcool, procédé qui consiste à faire réagir :
   (a) un alkyne-diol et/ou un alcène-diol dont les groupes alcool sont séparés par au moins deux atomes de carbone liés respectivement par la liaison alkynique ou alcénique, et
   (b) l'hydrogène
   en présence d'une composition catalytique à base d'un composé de palladium cationique, d'un anion de coordination et d'un ligand bidenté de formule générale :
   $R^1R^2-M-R-M-R^3R^4$
   dans laquelle M représente le phosphore, l'arsenic ou l'antimoine, R est un groupe organique bivalent de pontage contenant de 2 à 4 atomes de carbone dans le pontage et $R^1$, $R^2$, $R^3$ et $R^4$ représentent des groupes hydrocarbyle qui peuvent être ou ne pas être substitués par des groupes polaires.

2. Procédé selon la revendication 1, dans laquelle les groupes $R^1$, $R^2$, $R^3$ et $R^4$ représentent des radicaux aryle de 6 à 14 atomes de carbone.

3. Procédé selon la revendication 2, dans lequel les groupes aryle sont des groupes phényle.

4. Procédé selon l'une quelconque des revendications 1 à 3, dans lequel le groupe de pontage R est choisi parmi les groupes $-(CH_2)_n-$ , dans lequel n est 2, 3 ou 4 ; $-CH_2-C(CH_3)(CH_2MR^5R^6)-CH_2-$ dans lequel $R^5$ et $R^6$ ont les mêmes significations que $R^1$, $R^2$, $R^3$ et $R^4$ et M a la même signification que dans la revendication 1 ; o-phénylène et cis-vinylène.

5. Procédé selon l'une quelconque des revendications 1 à 4, dans lequel M représente le phosphore.

6. Procédé selon les revendications 3, 4 et 5, dans lequel le ligand est 1,3-bis(diphénylphosphino)-propane.

7. Procédé selon l'une quelconque des revendications 1 à 6, dans lequel le composé de palladium est à base d'un anion de coordination tel qu'un acétate.

8. Procédé selon l'une quelconque des revendications 1 à 7, dans lequel on utilise le composé de palladium en une proportion de 0,1 à 10 mole(s) par mole de ligand.

9. Procédé selon l'une quelconque des revendications 1 à 8, dans lequel le composé de palladium est présent à raison de $10^{-7}$ à $10^{-2}$ mole par mole de diol insaturé.

10. Procédé selon l'une quelconque des revendications 1 à 9, dans lequel outre le composé de palladium et le ligand, la composition du catalyseur comprend une base.

11. Procédé selon l'une quelconque des revendications 1 à 10, qu'on exécute à une température de 15 à 125°C et sous une pression d'hydrogène allant de l'atmosphérique à 100 bars.

12. Procédé selon l'une quelconque des revendications 1 à 11, qu'on exécute dans un milieu polaire tel que l'éther diméthylique de diéthylène-glycol.